# EUROPEAN PATENT SPECIFICATION

(11) **EP 2 402 439 B1**
(45) Date of publication and mention of the grant of the patent: **12.12.2018**
(21) Application number: 10746350.7
(22) Date of filing: 26.02.2010
(51) Int. Cl.: C12N 15/02, C12N 5/10, C12P 21/08, G01N 33/531, C07K 16/00, C07K 16/36, A61K 39/00

(54) **A METHOD FOR PREPARING A HYBRIDOMA CELL BY TRANSPLANTING BREAST CANCER CELLS**
VERFAHREN ZUR HERSTELLUNG EINER HYBRIDOMZELLLINIE DURCH TRANSPLANTATION VON BRUSTKREBSZELLEN
PROCÉDÉ DE PRÉPARATION D'UN HYBRIDOME PAR LA TRANSPLANTATION DE CELLULES DE CANCER DU SEIN

(30) Priority: 27.02.2009 JP 2009046730
(43) Date of publication of application: 04.01.2012
(73) Proprietor: Order-made Medical Research Inc., Kashiwa-shi Chiba 277-0882 (JP)
(72) Inventor: SATOFUKA, Hiroyuki, Nagareyama-shi Chiba 270-0101 (JP); UCHINO, Masahiro, Nagareyama-shi Chiba 270-0101 (JP); KOJIMA, Hiroko, Nagareyama-shi Chiba 270-0101 (JP)
(74) Representative: J A Kemp
(86) International application number: PCT/JP2010/053168
(87) International publication number: WO 2010/098471

(56) References cited:
- EP-A2- 0 280 209
- WO-A1-2005/111208
- WO-A2-2004/056847
- JP-A- 1 029 400
- JP-T- 2000 500 653
- JP-T- 2001 520 011
- US-A1- 2004 141 913
- OHNO YOSHIYA ET AL: "Production and characterization of highly tumor-specific rat monoclonal antibodies recognizing the extracellular domain of human l-type amino-acid transporter 1", CANCER SCIENCE, JAPANESE CANCER ASSOCIATION, TOKYO, JP, vol. 99, no. 5, 1 May 2008 (2008-05-01), pages 1000-1007, XP002476425, ISSN: 1347-9032, DOI: 10.1111/J.1349-7006.2008.00770.X
- STARLING J J ET AL: "MONO CLONAL ANTIBODIES TO HUMAN PROSTATE AND BLADDER TUMOR ASSOCIATED ANTIGENS", CANCER RESEARCH, vol. 42, no. 8, 1982, pages 3084-3089, XP009173416, ISSN: 0008-5472
- KITAMURA ET AL: "Specificity analysis of blood group Lewis-y (Le(y)) antibodies generatedagainst synthetic and natural Le(y) determinants.", PROCEEDINGS OF THE NATIONAL ACADEMY OF SCIENCES, vol. 91, no. 26, 1 December 1994 (1994-12-01), pages 12957-12961, XP55057081, ISSN: 0027-8424
- TOYA NATH BARAL ET AL: "Isolation of functional single domain antibody by whole cell immunization: Implications for cancer treatment", JOURNAL OF IMMUNOLOGICAL METHODS, ELSEVIER SCIENCE PUBLISHERS B.V.,AMSTERDAM, NL, vol. 371, no. 1, 16 June 2011 (2011-06-16) , pages 70-80, XP028254103, ISSN: 0022-1759, DOI: 10.1016/J.JIM.2011.06.017 [retrieved on 2011-06-30]
- HIROKO KOJIMA ET AL.: 'Ko Ten'isei Saibo Kabu MCF7-14 Juritsu Oyobi Kaiseki-Gan Shinjun-Gan Ten'i Kanren Inshi no Tansaku' DAI 80 KAI ANNUAL MEETING OF THE JAPANESE BIOCHEMICAL SOCIETY, DAI 30 KAI ANNUAL MEETING OF THE MOLECULAR BIOLOGY SOCIETY OF JAPAN, GODO TAIKAI KOEN YOSHISHU 2007, pages ABSTRACT - 3T18-12, XP008166771
- VIALE ET AL: "Pathological definitions of invasion, metastatic potential and responsiveness to targeted therapies", BREAST, EDINBURGH, GB, vol. 16, 1 January 2007 (2007-01-01), pages 55-58, XP022516919, ISSN: 0960-9776, DOI: 10.1016/J.BREAST.2007.07.007
- C. Khanna: "Modeling metastasis in vivo", Carcinogenesis, vol. 26, no. 3, 9 September 2004 (2004-09-09), pages 513-523, XP055407013, DOI: 10.1093/carcin/bgh261
- Anna Fantozzi ET AL: "Mouse models of breast cancer metastasis", BREAST CANCER RESEARCH, vol. 8, no. 4, 1 August 2006 (2006-08-01), XP055406995, GB ISSN: 1465-5411, DOI: 10.1186/bcr1530
- Tracy Vargo-Gogola ET AL: "Modelling breast cancer: one size does not fit all", NATURE REVIEWS. CANCER, vol. 7, no. 9, 1 September 2007 (2007-09-01), pages 659-672, XP055407044, GB ISSN: 1474-175X, DOI: 10.1038/nrc2193
- Z. Lu ET AL: "Epidermal Growth Factor-Induced Tumor Cell Invasion and Metastasis Initiated by Dephosphorylation and Downregulation of Focal Adhesion Kinase", MOLECULAR AND CELLULAR BIOLOGY., vol. 21, no. 12, 15 June 2001 (2001-06-15) , pages 4016-4031, XP55407745, US ISSN: 0270-7306, DOI: 10.1128/MCB.21.12.4016-4031.2001
- Yohko Kaneko ET AL: "A KINETIC ASPECT OF THE ANTIBODY PRODUCTION AGAINST EHRLICH TUMOR CELLS IN MICE BEARING EHRLICH SOLID TUMOR*1", , 1 October 1975 (1975-10-01), pages 573-575, XP55360269, Retrieved from the Internet: URL:https://www.jstage.jst.go.jp/article/c ancersci1959/66/5/66_5_573/_pdf
- Anne Tax ET AL: "Monoclonal antibodies against antigens displayed on a progressively growing mammary tumor (tumor-specifie antigens/mouse mammary tumor virus)", , 1 January 1981 (1981-01-01), pages 529-533, XP55360374, Retrieved from the Internet: URL:https://www.ncbi.nlm.nih.gov/pmc/artic les/PMC319087/pdf/pnas00652-0552.pdf [retrieved on 2017-03-30]

## Description

### TECHNICAL FIELD

The present invention relates to an immunization method using cancer cells.

### BACKGROUND ART

Since polyclonal and monoclonal antibodies have the ability to specifically bind to trace proteins contained in a mixed solution such as blood, they are industrially useful materials as reagents in researches and/or clinical tests or as pharmaceutical preparations.

Antibody molecules strongly induce antigen-antibody reaction against antigens expressed on the cell membrane, and hence desired antibodies are those recognizing membrane proteins in order to expect a therapeutic effect when administered to diseased patients.

Antibodies which have been marketed for use in disease treatment are mostly monoclonal antibodies recognizing membrane proteins (Non-patent Document 1), and they produce a therapeutic effect through their actions such as (1) binding to a target cell to induce cell death directly or indirectly (Patent Document 1) or (2) inhibition of ligand binding to membrane receptors to reduce intracellular signaling (Patent Document 2), etc.

Antibodies which have been marketed are each obtained by repeating inventive efforts and enormous works, and no simple method has been established for preparation of monoclonal antibodies against membrane proteins.

Various attempts have been made to develop techniques for obtaining antibodies recognizing membrane proteins.

For example, there is known a method in which a peptide sequence exposed on the cell membrane surface is prepared by forced expression in *E. coli* or other cells or by chemical synthesis, and the peptide sequence thus prepared is attached to a carrier protein to thereby induce immune responses.

However, such a method is disadvantageous in that it fails to cause three-dimensional structure formation and/or post-translational modifications (e.g., with sugar chains) and hence cannot yield antibodies recognizing the inherent structure of membrane proteins.

To obtain monoclonal antibodies against membrane proteins, the membrane proteins should retain their inherent three-dimensional structure during immunization into laboratory animals. However, when a surfactant is used for extraction of membrane proteins from the cell membrane, the membrane proteins lose their three-dimensional structure. Or alternatively, when no surfactant is used for this purpose, the membrane proteins aggregate through their hydrophobic regions. Because of these problems, it is not easy to prepare membrane proteins retaining their three-dimensional structure for use as antigens.

As a technique for full-length protein immunization, a genetic immunization method is reported, in which a DNA vector for expression is directly introduced into mice (Patent Document 3).

However, although such a method has many advantages, particularly in that it requires no purified antigen and in that it yields antibodies recognizing the three-dimensional structure of a target molecule, many disadvantages are also pointed out, for example, (1) immune responses cannot be induced unless the transgene is expressed on the cell surface, (2) sufficient immunization cannot be achieved due to low expression level of the transgene, (3) if the extracellular region is too small, the immune system cannot respond and no antibody is produced, and (4) it is difficult to obtain antibodies against multi-transmembrane proteins among membrane proteins (Non-patent Document 2).

In another method reported, a functional membrane protein is reconstructed on the baculovirus membrane and used for immunization (Non-patent Document 3).

However, it is reported in the document that this method causes weak immune responses during induction of antibody specific to a target molecule. Moreover, such a method has additional problems in that it results in post-translational modifications (e.g., addition of complex N-linked sugar chains) different from those found in mammalian cells because baculovirus is prepared in insect cells, or in whether all membrane proteins can take their functional structure on the viral membrane. These problems remain unchecked.

To eliminate the need for considering the problems of three-dimensional structures, some methods are reported, which involve direct administration of grown cells to mouse tail vein or abdominal cavity to cause immune responses (Patent Documents 4 to 6 and Non-patent Documents 4 to 8). In these methods, 1 to 10 × 10⁶ cells (corresponding to about 4 to 40 mg protein), which are larger than the normal antigen dose (100 to 200 µg target protein), are administered for a short period of time (at intervals of 1 week) and antibody-producing cells are collected from 1 to 6 weeks after the initiation of immunization.

For cell administration, an improved method is reported in which human cancer tissue is administered under the skin or into the gonadal fat pad in mice (Patent Document 7).

Patent Document 8 discloses a method for producing patient cancerous disease modifying antibodies using a novel paradigm of screening.

### Prior Art Documents

### Patent Documents

Patent Document 1: JP 2001-010974 A
Patent Document 2: JP 07-188056 A
Patent Document 3: JP 07-313187 A
Patent Document 4: WO2006/109533
Patent Document 5: JP 06-327491 A
Patent Document 6: JP 2004-517885 A
Patent Document 7: JP 2001-520011 A
Patent Document 8: US 2004/0141913

### Non-patent Documents

Non-patent Document 1: Folia Pharmacol. Jpn 2008 131 p. 102-108
Non-patent Document 2: Seibutsu Kogaku (Biotechnology) 2008 86(8) p. 384-386
Non-patent Document 3: J. Immunol. Methods 2007 322 (1-2) p. 104-117
Non-patent Document 4: Somatic Cell Genet. 1979 5(6) p. 957-971
Non-patent Document 5: J Clin. Invest. 1981 68(5) p. 1331-1337
Non-patent Document 6: Cancer Res. 1986 46(10) p. 5137-5143
Non-patent Document 7: Cancer Res. 1982 42(2) p. 601-608
Non-patent Document 8: Proc. Natl. Acad. Sci. USA 1979 76(3) p. 1438-1442
Non-patent Document 9: Antibodies: A Laboratory Manual 1988 Chapter 5 p. 114-115

### DISCLOSURE OF THE INVENTION

### PROBLEM TO BE SOLVED BY THE INVENTION

The methods disclosed in Patent Documents 4 to 6 and Non-patent Documents 4 to 8 have the following problems: (1) immune responses are difficult to occur against a specific substance because many kinds of proteins are immunized at the same time, (2) anaphylactic shock will be caused because proteins are administered in large amounts, and (3) animals are more likely to die earlier, e.g., due to organ failure caused by metastasis of cancer cells.

Moreover, to obtain many types of high-affinity antibodies, it is important to ensure the progress of hyperimmunization. In conventional methods, it is known that immunization with a lower antigen dose for a longer period of time is more likely to facilitate hyperimmunization. For example, in the case of mice, multiple administrations (empirically 5 or more) at intervals of 3 weeks or longer are known to be preferred (Non-patent Document 9). Hyperimmunization refers to a combination of the following three mechanisms: class shift to IgG, affinity maturation, and clonal dominance (apoptosis-induced selective death of antibody-producing cells with low specificity), and it is known as a mechanism which allows selective proliferation of high-affinity antibodies (Non-patent Document 9).

In the methods disclosed in Patent Documents 4 to 6 and Non-patent Documents 4 to 8, mice are more likely to die earlier and hyperimmunization is less likely to proceed, because a large amount of cells are administered for induction of immune responses. Thus, the methods for direct cell administration disclosed in these documents cannot establish conditions sufficient to induce effective immune responses.

The method disclosed in Patent Document 7 has problems in that (1) it is necessary to isolate human tissues containing B cells, (2) immune responses are not reproducible due to differences in the types of cells contained in the tissues, and (3) *in vitro* culture is difficult and gene transfer or other techniques are also difficult to apply.

In view of the foregoing, there is a demand for the establishment of a simple method for preparing antibodies against membrane proteins.

Thus, the object of the present invention is to provide an immunization method which is intended to overcome the problems stated above and which allows efficient preparation of a desired antibody.

### MEANS TO SOLVE THE PROBLEM

As a result of extensive and intensive efforts made to achieve the above object, the inventors of the present invention have found that a desired antibody can be obtained efficiently when cancer cells having a metastatic potential are transplanted into and engrafted in laboratory animals. This finding led to the completion of the present invention.

Namely, the present invention is as follows.
[1] A method preparing a hybridoma cell, which comprises the following steps:
   (1) transplanting breast cancer cells into breast, mammary gland, bone, lung or lymph node of a laboratory animal, wherein the breast cancer cells are at least one selected from the group consisting of MCF7-14, MDA-MB231, MDA-MB361 and MDA-MB435;
   (2) keeping the animal for 10 weeks or longer to engraft the breast cancer cells in the laboratory animal; and
   (3) collecting antibody-producing cells from the immunized laboratory animal to fuse them with myeloma cells, thereby preparing a hybridoma cell.
[2] The method according to [1] above, wherein the organ is breast or mammary gland.
[3] The method according to [2] above, wherein the wherein the mammary gland is the fourth mammary gland.
[4] The method according to any one of [1] to [3] above, wherein the laboratory animal is a rodent.
[5] The method according to any one of [1] to [4] above, wherein the laboratory animal is an immunodeficient animal.
[6] The method according to any one of [1] to [5] above, wherein the laboratory animal is a BALB/c-nu/nu nude mouse.
[7] The method according to any one of [1] to [6] above, wherein a scaffold material is used for transplantation in step (1).
[8] The method according to [7] above, wherein a component of the scaffold material is at least one selected from the group consisting of laminin, entactin, collagen, fibrin, agarose, polyvinyl alcohol, polyethylene glycol, polylactic acid, and polyglycolic acid.
[9] The method according to [8] above, wherein the scaffold material is Matrigel.
[10] A method for preparing a monoclonal antibody, which comprises the following steps:
   (1) preparing a hybridoma cell by a method as defined in any one of [1] to [9] above; and
   (2) culturing the hybridoma cells to obtain the desired antibody.
[11] A method according to [10] above, wherein the antibody binds to a membrane protein.

### EFFECT OF THE INVENTION

The present invention enables the provision of a novel method by which a desired hybridoma cell or antibody can be obtained efficiently.

### BRIEF DESCRIPTION OF THE DRAWINGS

Figure 1 shows the results analyzed for the titers of antibodies contained in plasma. Mouse group A is a group receiving MCF7 transplantation, mouse group B is a group receiving MCF7-14 transplantation, and mouse group C is an unimmunized group. Mouse groups A and B show reactivity with the transplanted cells, whereas mouse group C shows reactivity with both MCF7 and MCF7-14.
Figure 2 shows a photograph of excised spleens on a culture dish of 6 cm diameter. This photograph shows spleens from the unimmunized group and the group receiving MCF7-14 transplantation.
Figure 3 shows the results calculated for the number of spleen cells, in which each error bar represents standard deviation.
Figure 4 shows images of immunostaining observed under a phase contrast microscope (1) and under a fluorescent microscope (2).
Figure 5 shows the results confirmed for antigen protein expression in cells. The vertical axis represents the reactivity between F3 gene-transformed cells (1 to 7) or non-transformed cells (Negative control) and F3-recognizing antibody.
Figure 6 shows the results analyzed for the titers of antibodies contained in hybridoma culture supernatants, in comparison with medium alone (Negative control). The vertical axis represents the reactivity of each antibody to F3 protein.

### BEST MODES FOR CARRYING OUT THE INVENTION

The present invention will be described in more detail below with respect to preferred embodiments.

The method of the present invention is a method for preparing a hybridoma cell, which comprises the following steps:
(1) transplanting breast cancer cells having a metastatic potential and capable of expressing an antigen into the laboratory animal; and
(2) allowing the cancer cells to be engrafted in the laboratory animal to thereby immunize the laboratory animal; and
(3) collecting antibody-producing cells from the laboratory animal to fuse them with myeloma cells.

In the present invention, effective immunization is achieved when cancer cells having a metastatic potential and capable of expressing an antigen are transplanted into and engrafted in laboratory animals. According to the present invention, it is possible to obtain many types of antibody-producing cells in which hyperimmunization has proceeded sufficiently. Moreover, the present invention allows induction of higher immune responses when compared to conventional immunization methods.

Hereinafter, the "breast cancer cells having a metastatic potential and capable of expressing an antigen" are also simply referred to as "cancer cells having a metastatic potential."

In the present invention, by allowing cancer cells having a metastatic potential to be engrafted and directly used for immunization, antigens can be immunized while retaining their functional three-dimensional structure and post-translational modifications, and antibodies against membrane proteins can be induced, which have been difficult to obtain. Moreover, in the present invention, antibodies can be obtained against antigens having their inherent three-dimensional structure (which means their structure found in an *in vivo* environment), so that it is possible to induce antibodies having sufficient neutralizing capacity against the antigens. Further, in the present invention, antibodies are induced not only against membrane proteins, but also against proteins in the cytoplasm and nuclei. Thus, the present invention has a high utility value as an immunization method for preparing a hybridoma cell. Induction of immune responses against intracellular substances appears to be mediated by a mechanism in which macrophages or other cells decompose the transplanted cancer cells by their phagocytic action to present the intracellular substances as antigens.

As used herein, the phrase "having a metastatic potential" is intended to mean that metastatic ability is observed when assayed by a method used to evaluate the metastatic ability of cells. For example, in a case where cancer cells are transplanted into laboratory animals, a state that the cancer cells form distant metastasis is expressed as "having a metastatic potential."

As used herein, the phrase "having a high metastatic potential" is intended to mean that metastatic ability is observed at a high percentage when assayed by a method used to evaluate the metastatic ability of cells. For example, in a case where cancer cells are transplanted into laboratory animals, a state that the cancer cells form distant metastasis in at least 20% or more, preferably 40% or more, more preferably 60% or more of the laboratory animals receiving transplantation is expressed as "having a high metastatic potential."

As used herein, the term "distant metastasis" is intended to mean metastasis to an organ located far away from the primary focus.

As used herein, the phrase "capable of expressing an antigen" is intended to mean containing an antigen in an amount sufficient to induce immune responses.

As used herein, the term "antigen" is intended to mean a substance capable of inducing immune responses, which is not limited in any way as long as it is expressed in cancer cells to be transplanted and causes antigen-antibody reaction. Examples of an antigen available for use include proteins, nucleic acids, peptides, sugars, and lipids. A preferred antigen is a protein, which may be either a native protein expressed in cancer cells or a recombinant protein expressed by introduction of any gene into cells. Introduction of any gene into cells may be accomplished in a known manner, for example, by using a vector such as a plasmid. In this case, sugar chains added to the protein may also serve as antigens. It is possible to use either a single or several antigens. In either case, protein molecules are localized at any site in cells, for example, in the nuclei, in the cytoplasm, on the cell membrane, etc.

Likewise, the size of antigen is not limited in any way. When a protein is expressed as an antigen in cancer cells of the same animal origin and used for transplantation, it is preferred because the protein can stimulate immune responses while retaining its inherent three-dimensional structure and modifications (e.g., with sugar chains).

The method uses breast cancer cells having a high metastatic potential. The breast cancer cells used in the method of the invention are at least one selected from MCF7-14 derived from MCF7, which was established from human breast cancer (WO2008/093886, Accession No. FERM BP-10944), MDA-MB231, MDA-MB361 and MDA-MB435. Other cancer cells having a high metastatic potential include HT-1080 (fibrosarcoma), HepG2 (liver cancer), T-24 (urinary bladder cancer), SW620 (large bowel cancer), A549 (lung cancer), SW480 (colon cancer) andA375 (melanoma).

In a case where the transplantation site of cancer cells having a metastatic potential is the proximity of mammary glands, preferred are breast cancer cells expressing one or more proteins selected from estrogen receptor, progesterone receptor and Her-2. Breast cancer cells expressing these proteins are more likely to proliferate in mammary glands and hence are effective in engraftment after transplantation.

Cancer cells having a metastatic potentialinclude not only cells inherently having a metastatic potential, but also cancer cells prepared to have a metastatic potential through any means conceived by those skilled in the art, including introduction of a gene, administration of growth factor (e.g., hormone or cytokine), induction of a genetic mutation by UV irradiation or drug treatment, or isolation of cells carrying a spontaneous mutation or an acquired change in chromatin modifications.

Any laboratory animals may be transplanted with cancer cells having a metastatic potential as long as the transplanted cells are engrafted in the animals to cause humoral immune responses in the animals.

Although such laboratory animals may be of any type, they are preferably rodents such as mice and rats, more preferably immunodeficient rodents, and even more preferably immunodeficient mice.

As laboratory animals, inbred or closed colony immunodeficient mice can be used. Specific examples of immunodeficient mice include BALB/c, C57BL/6 and ICR immunodeficient mice. Among them, female BALB/c-nu/nu mice are preferred because their reduced T cell functions facilitate engraftment of the transplanted cells and also because they are easy to keep.

As used herein, the term "transplanting," "transplanted" or "transplantation" is intended to mean that cancer cells are transferred to and implanted in laboratory animals, and it excludes direct administration of cancer cells into the blood or abdominal cavity.

In the step of transplanting cancer cells having a metastatic potential into a laboratory animal, the cancer cells can be transplanted into the laboratory animal in a conventionally known manner.

To transplant cancer cells having a metastatic potential, the cells may be injected into laboratory animals not only in the form of a cell suspension, but also in the form of cells cultured into a two- or three-dimensional structure, such as a sheet form or a multi-layered structure.

Although there is no particular limitation on the number of cancer cells having a metastatic potential to be used for transplantation, it is preferably at least 0.1 × 10⁶ cells, and it is desirable to use as much cells as possible within the range sufficient to effectively induce immune responses.

As to the number of cells used in conventional immunization methods via the tail vein or intraperitoneal route, 1 × 10⁶ cells to 1 × 10⁷ cells are required for the primary immunization and each booster. In the present invention, at least 0.1 × 10⁶ cells may be used only for transplantation, and hence immune responses can be induced by a smaller number of cells than in the conventional methods.

For improved engraftment of cancer cells having a metastatic potential, it is desirable to inject a cell suspension in admixture with a scaffold material.

Any scaffold material may be used for this purpose as long as it serves as a scaffold for the growth of cancer cells to be engrafted. Preferred examples include gels, hydrogels and resins, which are composed of laminin, entactin, collagen, fibrin, agarose, polyvinyl alcohol, polyethylene glycol, polylactic acid, polyglycolic acid and so on as components.

As a scaffold material, a gel comprising laminin/entactin and collagen as its major components is preferred in terms of the hardness of the formed gel and/or good proliferation of the transplanted cells, with Matrigel (Becton Dickinson) being more preferred. Preferred examples of Matrigel include growth factor reduced Matrigel as disclosed in J. Steroid. Biochem. Molec. Biol. 1993 44(4-6) p. 671-673.

The transplantation site of cancer cells having a metastatic potential is an organ or tissue in which the transplanted cells are easily engrafted.

Examples of an organ into which cancer cells having a metastatic potential are transplanted include an organ to which the primary focus of cancer from which such cancer cells are isolated belongs, and organs to which metastatic lesions belong. In the present invention, transplantation into an organ to which the primary focus belongs is referred to as orthotopic transplantation, while transplantation into organs to which metastatic lesions belong is referred to as heterotopic transplantation.

Organs to which metastatic lesions belong are intended to include actual organs for which the formation of metastatic lesions from the cancer cells have been reported, as well as possible organs for which distant metastasis will be presumed in cancer of the same type from statistical data.

Orthotopic transplantation into the breast is possible, but it is also possible to use heterotopic transplantation into other organs in which metastasis occurs frequently, as exemplified by bone, lung and lymph node. The organ receiving transplantation is preferably intended for orthotopic transplantation, and as the cells are breast cancer cells, it is preferably the breast or mammary gland.

In the present invention, even if the animal species from which cancer cells are isolated is different from the animal species into which the cancer cells are transplanted, transplantation into an organ having the same functions falls within orthotopic transplantation.

As a tissue into which cancer cells having a metastatic potential are transplanted, preferred is the same tissue as that in which the primary focus was formed. Other preferred examples are tissues in the proximity of lymph nodes.

The tissue into which cancer cells having a metastatic potential are transplanted may be a tissue within the organ to which the primary focus belongs or the organs to which metastatic lesions belong, as in the case of the same tissue as that in which the primary focus was formed, or alternatively, may be a tissue outside the organ to which the primary focus belongs or the organs to which metastatic lesions belong, as long as cancer cells having a metastatic potential are engrafted in such a tissue.

As used herein, the phrase "the proximity of lymph nodes" is intended to mean the periphery of lymph nodes, preferably adjacent to lymph nodes.

Although the proximity of lymph nodes is not limited in any way, in the case of mice, it may be within 1 cm, preferably within 5 mm, and more preferably within 1 mm from the lymph node, by way of example. In the proximity of lymph nodes, the engrafted cells will be easily supplied to the lymph nodes, which facilitate stimulation of immune responses. Thus, the proximity of lymph nodes is preferred.

As used herein, the term "fat tissue" is intended to mean a tissue composed of fat cells. Examples of a tissue covered with fat tissue include mammary glands.

Fat tissue is suitable as a transplantation site of cancer cells because this tissue is easy to surgically manipulate for transplantation and allows easy engraftment of cells. In a case where mice are used as laboratory animals for transplantation of breast cancer cells, the cells are preferably transplanted into the fourth mammary gland. The fourth mammary gland is not only located in the proximity of lymph nodes and covered with fat tissue, but is also particularly easy to manipulate for transplantation among mammary glands and allows long-term engraftment.

The transplantation site is not limited to the organ to which the primary focus of cancer cells belongs or the organs to which metastatic lesions belong.

As used herein, the term "engrafted" or "engraftment" is intended to mean that the transplanted cancer cells stay at the site and proliferate to form cell aggregates.

For engraftment of cancer cells having a metastatic potential within laboratory animals, it is sufficient to keep these laboratory animals for a period of time during which immune responses occur, after transplantation of the cancer cells. Boosters, which are used in standard immunization, are not required for this purpose. Once the cancer cells having a metastatic potential are transplanted into the laboratory animals, it is preferred that no booster is used for engraftment within the laboratory animals.

The reason why the present invention does not include any booster step would be because since the transplanted cancer cells have a metastatic potential, cancer cells having a metastatic potential may be constantly supplied from the engrafted site to lymph nodes or blood vessels to thereby continuously stimulate immune responses.

To obtain monoclonal antibodies with high affinity or specificity to antigens, it is important to ensure the progress of hyperimmunization. Thus, the period for keeping laboratory animals in the step of engraftment and immunization is preferably as longer as possible within the range where the animals can survive.

The indicators of hyperimmunization include the size of spleen and the number of spleen cells. Since the size of spleen and the number of spleen cells reflect the degree of proliferation of antibody-producing cells, the variety of antibody-producing cells will increase with increase in the number of spleen cells. Namely, a larger number of spleen cells would allow production of antibodies with more improved performance.

If the laboratory animals are mice, the number of cells obtained from a non-sensitized mouse spleen is 0.5 to 1 × 10⁸ cells. Upon immunization using an adjuvant or the like, it is known that the number of cells is increased up to 2 to 4 × 10⁸ cells by repeated administration for 15 weeks, and hyperimmunization proceeds in such immunized mice. It is shown that antibodies having a certain affinity and specificity to desired antigens are obtained when the number of spleen cells is 3 × 10⁸ cells or more.

In the present invention, a small amount of cells are sufficient to achieve immunization, which prevents early death of laboratory animals.

In conventional methods in which cells are administered to the abdominal cavity or tail vein for immunization, the administered cells are more likely to disappear earlier due to the phagocytic action of macrophages or the like. Moreover, when cancer cells having a metastatic potential are administered to mice via the tail vein, the mice will die within a short period of time (within 1 to 6 weeks after transplantation) as a result of sudden stimulation of immune responses. In some actual cases, all mice died within 7 weeks after administration (Toagosei annual report TREND 1999 vol. 2, p. 32).

In the present invention, many mice receiving transplantation have been confirmed to survive over a long period of at least 10 weeks or longer, usually 15 weeks or longer. Further, since the present invention allows continuous supply of cancer cells, immune responses are stimulated continuously, hyperimmunization proceeds efficiently, and the number of spleen cells can be increased to 9 to 13 × 10⁸ cells.

In the present invention, hyperimmunization may proceed within a short period of time in some cases, depending on the type of antigen or cell to be transplanted, the number of cells to be transplanted, etc. In such cases, the keeping of the animals may be stopped earlier to collect antibody-producing cells as described later.

A preferred antigen to be expressed by cancer cells having a metastatic potential is a protein, and examples of a desired protein include, but are not limited to, membrane proteins.

The present invention is also directed to a method for preparing a monoclonal antibody, which comprises the following steps:
(1) transplanting cancer cells having a metastatic potential and capable of expressing an antigen into a laboratory animal;
(2) allowing the cancer cells to be engrafted in the laboratory animal to thereby immunize the laboratory animal;
(3) collecting antibody-producing cells from the laboratory animal to fuse them with myeloma cells, thereby preparing hybridoma cells; and
(4) culturing the hybridoma cells.

In the present invention, after the step of allowing cancer cells having a metastatic potential to be engrafted in a laboratory animal to thereby immunize the laboratory animal, monoclonal antibodies can be obtained in any manner well known to those skilled in the art.

Antibody-producing cells are collected from the spleen or lymph nodes of the immunized laboratory animal, and fused with myeloma cells to prepare hybridoma cells, followed by screening to select hybridoma cells producing monoclonal antibodies specific to a desired antigen, preferably a desired protein.

By culturing these hybridoma cells, the monoclonal antibodies can be prepared stably.

According to the present invention, high immune responses can be induced, and hence the immunization method of the present invention is very advantageous in the establishment of hybridoma cells producing high-affinity antibodies.

### EXAMPLES

The present invention will be further described in more detail by way of the following examples and comparative examples, which are not intended to limit the technical scope of the invention.

### Example 1. Preparation of antibodies against membrane proteins

### (1) Cells

MCF7 and MDA-MB231 were purchased from the Institute of Development, Aging and Cancer, Tohoku University (TKG 0479) and the American Type Culture Collection (ATCC, Manassas, VA, USA), respectively.

MCF7-14 was obtained under Accession No. FERM BP-10944 and subcultured before use.

MCF7, MCF7-14 and MDA-MB231 were each cultured and subcultured at 37°C under 5% CO₂ for 48 to 72 hours in RPMI1640 medium (Sigma) containing 10% (v/v) serum (EQUITECH-BIO), such that cell confluency did not exceed 80%.

### (2) Transplantation of cells

The proliferated cells were collected and washed twice with PBS(-) (0.01 M sodium-phosphate buffer, 0.138 M NaCl, 0.0027 M KCl, pH 7.4). The washed cells were suspended at a final density of 8.6 × 10⁷ cells/mL in growth factor reduced Matrigel (Becton Dickinson) and stored on ice before use in transplantation.

Chloral hydrate (Sigma) was dissolved at a concentration of 3.5% (w/v) in physiological saline to prepare a 3.5% solution of chloral hydrate in physiological saline. Nude mice at 6 to 8 weeks of age (BALB/cALcl-nu/nu line (CLEA Japan, Inc., Japan)) were anesthetized by being intraperitoneally administered with 0.2 mL of the 3.5% solution of chloral hydrate in physiological saline. Into the fourth mammary glands in each mouse, the cells suspended in the Matrigel were transplanted at 1 × 10⁶ cells per mammary gland via a 24G injection needle, such that the cells did not extend off the mammary gland. Each mouse received two transplantations, one at left and another at right fourth mammary gland in the trunk.

In this method, no booster was used, unlike standard immunization.

### (3) Collection of plasma fractions

After transplantation, the mice were kept for 15 weeks or longer. Cancer formation at each transplantation site was visually and palpably monitored over time. From the cancer cell-transplanted mice and the unimmunized mice, 20 µL of blood was collected via the tail vein. Each collected blood was mixed with 1 µL heparin solution (Ajinomoto Pharma Co., Ltd., Japan). The resulting mixture was centrifuged at 3000 × g for 5 minutes to precipitate the cells. The supernatant was collected as a plasma solution.

### (4) Analysis of immune responses

MCF7, MCF7-14 and MDA-MB231 cultured in (1) above were each seeded at 80% confluence in a 96-well plate and cultured at 37°C under 5% CO₂ for 16 hours.

After removal of the culture supernatant, 100 µL of a 10% (v/v) neutral buffered formalin solution (WAKO) was added and reacted for 10 minutes at room temperature. After removal of the formalin solution, each plate was washed three times with PBS(-) and then air-dried to give a plate in which the cells of each type were immobilized.

The plasma solutions collected in (3) above were each diluted 20,000-fold with TBS-T (25 mM Tris, 150 mM NaCl, 0.05% (v/v) Tween20, pH 7.4). As a primary antibody, each plasma dilution was added in a volume of 100 µL per well in the immobilized plates and reacted at room temperature for 1 hour. Each well was washed three times with 200 µL TBS-T.

As a secondary antibody, anti-mouse IgG polyclonal antibody-HRP label (BETHYL) was diluted 5,000-fold with TBS-T. This antibody dilution was added in a volume of 100 µL per well and reacted at room temperature for 30 minutes. Each well was washed three times with 200 µL TBS-T.

Orthophenylenediamine (Sigma) was diluted with 50 mM carbonate-citrate buffer (pH 5.0) to give a final concentration of 0.5 mg/mL, and mixed with 1/10,000 volumes of 35% (w/w) aqueous hydrogen peroxide (WAKO). This mixture was added as a substrate solution in a volume of 100 µL per well and reacted at room temperature for 10 minutes. 25 µL of 3 N sulfuric acid (WAKO) was added to stop the reaction. The absorbance at 492 nm was measured with a plate reader (SpectraMaxPure384, Molecular Devices) to analyze the titer in each plasma solution.

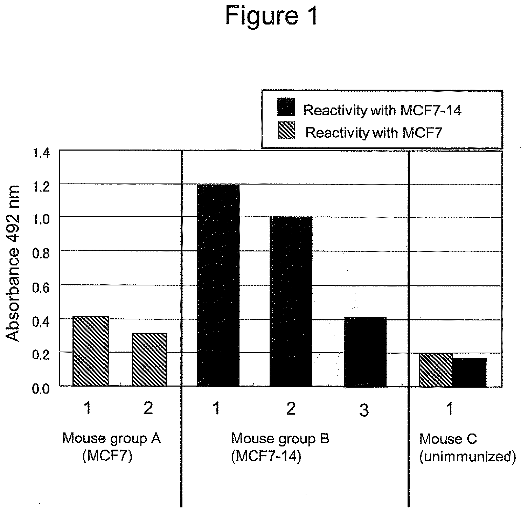

Figure 1 shows the results analyzed for the titers of antibodies contained in plasma of the MCF7- or MCF7-14-transplanted mice and unimmunized mice.

In comparison with the unimmunized mice, the mice transplanted with MCF7 having a metastatic potential showed antibody production, and the mice transplanted with more metastatic MCF7-14 clearly showed antibody production. In the mice transplanted with MDA-MB231 also showed antibody production (data not shown).

### (5) Spleen size and the number of leukocytes contained in spleen

The spleen is an organ responsible for proliferation and differentiation of B cells. When a foreign material enters the body and stimulates humoral immune responses, this organ plays a role in specifically increasing the number of B cells which produce antibodies recognizing the foreign material. Based on this fact, the number of leukocytes contained in the spleen can be used as one of the indicators for immune responses against an antigen.

From the mice transplanted with the cells in (2) above, spleen tissues were excised and compared for their size.

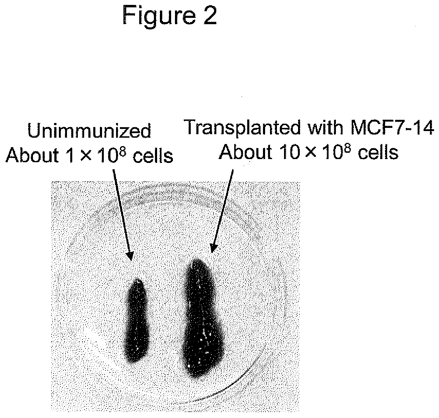

In comparison with the unimmunized spleens, the spleens of the MCF7-14-transplanted mice were clearly enlarged (Figure 2). The MDA-MB231-transplanted mice showed similar spleen enlargement, as in the case of MCF7-14 (data not shown).

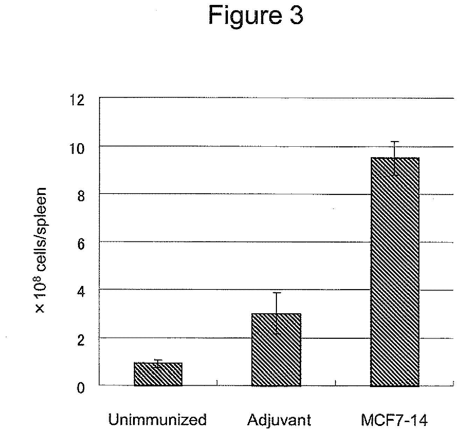

To calculate cell counts in each case, cells in each spleen were collected into RPMI1640 medium using an injection needle and a pair of tweezers to give a cell suspension. The cell suspension (100 µL) and Turk's solution (900 µL, Nacalai Tesque, Inc., Japan) were mixed together. The leukocyte concentration was measured with a hemacytometer (AS ONE Corporation, Japan) and used to calculate the number of leukocytes per spleen. As a result, the unimmunized mice had 0.8 to 1 × 10⁸ leukocytes per spleen, whereas the MCF7-14-transplanted mice had 10 × 10⁸ leukocytes, which increased about 10-fold. Figure 3 shows the number of spleen cells (the number of leukocytes per spleen) obtained from two unimmunized mice, 100 mice receiving standard immunization using adjuvants (e.g., Freund's complete adjuvant (PIERCE), incomplete adjuvant (PIERCE), Ribi adjuvant system (Funakoshi Co., Ltd., Japan), GERBU adjuvant (Nacalai Tesque, Inc., Japan)), and three mice transplanted with MCF7-14. In comparison with the immunization using adjuvants which resulted in up to 2 to 4 × 10⁸ cells, the method of the present invention was confirmed to induce high immune responses never before achieved.

### (6) Cell fusion

MCF7-14-derived mouse spleen lymphocytes were fused in a standard manner with mouse myeloma cell line P3X63-Ag8 (ATCC Accession No. CRL-1580) using 50% (v/w) polyethylene glycol 4000 (Sigma).

The fused cells were suspended in HAT medium (Invitrogen) and dispensed into twenty 96-well plates in a volume of 100 µL per well. During culture, 200 µL of HAT medium was added to each well. After culture for 11 to 16 days, the plates were observed under a microscope, indicating that 3 to 6 colonies were formed per well.

### (7) Analysis of hybridoma cells

From the 96-well plates showing the growth of hybridoma cells, the culture supernatants were collected in 200 µL volumes and analyzed for reactivity with cells in the same manner as shown in (4) above to screen antibodies capable of reacting with MCF7-14.

Among the resulting antibodies, five hybridoma cells were randomly selected and monocloned. These cells were cultured in HT medium (Invitrogen), followed by immunostaining against MCF7-14 using antibodies contained in the supernatants and FITC-labeled anti-mouse IgG (Becton Dickinson).

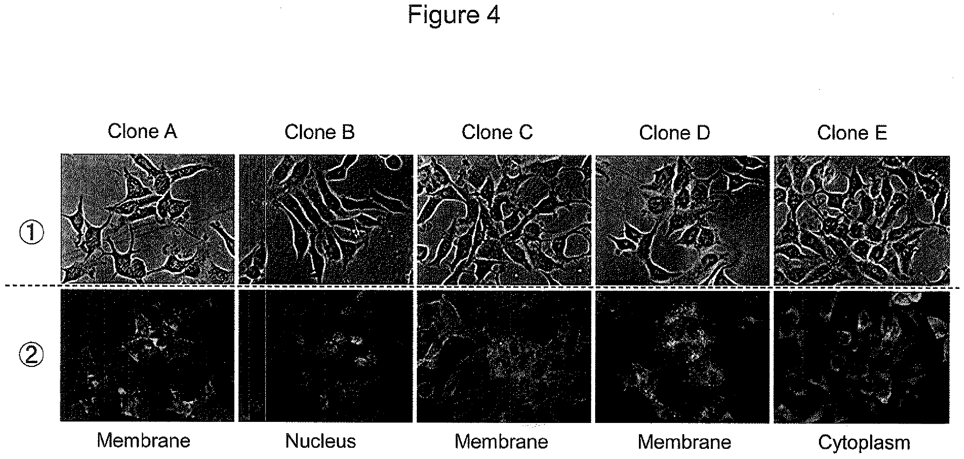

Figure 4 shows the results observed for cell morphology under a phase contrast microscope (1) and the results detected for the stained regions under a fluorescent microscope (2).

Among the antibodies obtained from the five types of hybridoma cells, three antibodies (clones A, C and D) were found to recognize cell membrane proteins, while the remaining antibodies were found to recognize proteins present in the nuclei (clone B) and the cytoplasm (clone E), respectively. The result showing that three (60%) of the five clones randomly selected were antibodies against membrane proteins strongly indicates that the immunization method of the present invention is a procedure allowing easy preparation of antibodies against membrane proteins, which have been difficult to prepare.

### Example 2. Preparation of antibodies against membrane protein (F3)

### (1) Antigen

Human tissue factor (coagulation factor III, F3), which is a membrane-bound protein, is a factor responsible for the initiation of extrinsic blood coagulation reaction. Antibodies against this membrane protein were prepared.

### (2) Construction of expression vector

RNA extracted from HeLa cells using an SV Total RNA Isolation System (Promega) was converted into cDNA using SuperScriptIII RNase H-Reverse Transcriptase (Invitrogen), and this cDNA was used as a template in PCR to amplify F3. The amplified fragment was cloned into pEF6/Myc-HisA (Invitrogen) such that the Tag sequence was deleted. Each manipulation was conducted in accordance with the document attached to the kit.

### (3) Gene expression on cells

Into MDA-MB231 described in Example 1(1), the plasmid constructed in (2) above was introduced using FUGENE6 (Roche Applied Science). The manipulation was conducted in accordance with the document attached to the kit. The cells were cultured in the same medium as shown in Example 1(1) supplemented with 10 µg/mL blasticidin S hydrochloride while repeating medium replacement every 3 to 5 days to select drug-resistant cells. To pick out cells showing forced expression of F3 from among the resulting resistant strains, the expression level of F3 was confirmed in the same manner as shown in Example 1(4) by cell-based enzyme immunoassay (ELISA). It should be noted that Anti Coagulation Factor 3/Tissue Factor (R&D systems), which is anti-F3 polyclonal antibody, was diluted to 1 µg/mL for use as a primary antibody. Peroxidase AffiniPure Goat anti-bovine IgG (H+L) (Jackson ImmunoResearch) was used as a secondary antibody. In addition, TMB+ (DAKO) was used as a substrate solution and the absorbance at 450 nm was measured with a plate reader to analyze antibody titers.

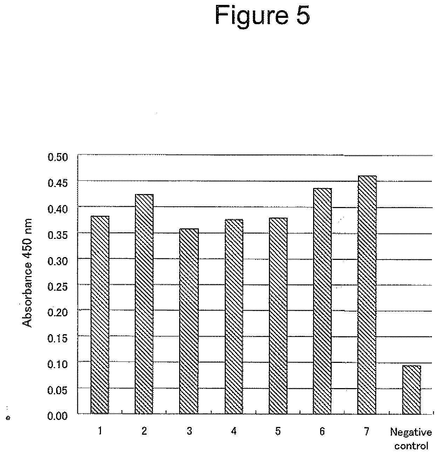

Figure 5 shows the results of expression analysis with anti-F3 polyclonal antibody performed on the F3 gene-transformed cells (1 to 7) and non-transformed cells (Negative control).

The transformed cells were found to show significantly increased F3 expression when compared to the non-transformed cells.

### (4) Transplantation of F3-expressing cells

Among these cells confirmed to express F3, five types of cells (1 to 5) showing substantially uniform growth were selected and cultured. The grown cells were collected with trypsin and washed twice with PBS(-). These five types of cells were mixed to a uniform state and then transplanted into mice in the same manner as shown in Example 1(2). In this method, no booster was used, unlike standard immunization.

### (5) Obtaining of monoclonal antibodies

At 7 months after transplantation, spleen cells were collected and used to prepare hybridoma cells in the same manner as shown in Example 1(6). To select antibodies recognizing F3, ELISA using F3 protein was performed as follows to analyze the titers of antibodies produced by the hybridoma cells.

A recombinant F3 protein (Coagulation Factor III/Tissue Factor, human, Recombinant, Carrier-free, R&D systems) was diluted to 2 µg/mL in PBS(-) and dispensed in 100 µL volumes into 96-well plates. After adsorption for 1 hour at room temperature, the plates were blocked for 30 minutes at room temperature with a solution prepared to contain 1% (w/v) bovine serum albumin in PBS(-). After washing with TBS-T, the same procedure as shown in Example 1(4) was repeated, except that antibody contained in each culture supernatant of hybridoma cells was used as a primary antibody and a solution of anti-mouse IgG polyclonal antibody-HRP label (BETHYL) diluted 5,000-fold in TBS-T was used as a secondary antibody.

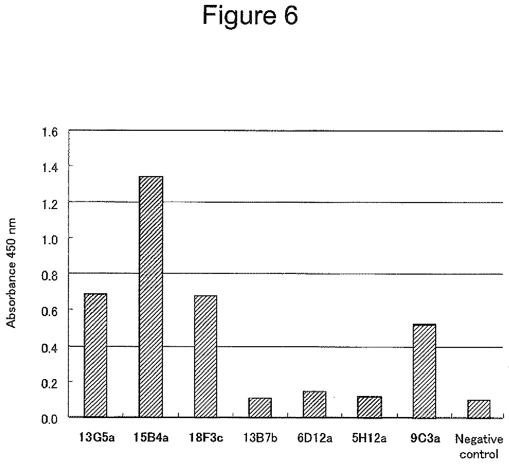

Figure 6 shows the results analyzed for the titers of antibodies produced by the hybridoma cells (indicated with their clone number), in comparison with medium alone (Negative control).

Against the membrane protein F3 expressed by cells, several monoclonal antibodies were obtained, as typified by clones 13G5a, 15B4a, 18F3c, 9C3a, etc.

### INDUSTRIAL APPLICABILITY

The present invention enables the preparation of antibodies which have been difficult to obtain by conventional methods. Moreover, the present invention also enables the preparation of antibodies against the inherent three-dimensional structure of antigens, and hence enables the provision of antibodies having sufficient neutralizing capacity against the antigens.

The present invention makes it possible to provide a new material on the market as a novel antibody for clinical laboratory tests or therapeutic purposes.

## Claims

1. A method for preparing a hybridoma cell, which comprises the following steps:
(1) transplanting breast cancer cells into breast, mammary gland, bone, lung or lymph node of a laboratory animal, wherein the breast cancer cells are at least one selected from the group consisting of MCF7-14, MDA-MB231, MDA-MB361 and MDA-MB435;
(2) keeping the animal for 10 weeks or longer to engraft the breast cancer cells in the laboratory animal; and
(3) collecting antibody-producing cells from the immunized laboratory animal to fuse them with myeloma cells,
thereby preparing a hybridoma cell.

2. The method according to claim 1, wherein the organ is breast or mammary gland.

3. The method according to claim 1 or 2, wherein the mammary gland is the fourth mammary gland.

4. The method according to any one of the preceding claims, wherein the laboratory animal is a rodent.

5. The method according to any one of the preceding claims, wherein the laboratory animal is an immunodeficient animal.

6. The method according to any one of the preceding claims, wherein the laboratory animal is a BALB/c-nu/nu nude mouse.

7. The method according to any one of the preceding claims, wherein a scaffold material is used for transplantation in step (1).

8. The method according to claim 7, wherein a component of the scaffold material is at least one selected from laminin, entactin, collagen, fibrin, agarose, polyvinyl alcohol, polyethylene glycol, polylactic acid, and polyglycolic acid.

9. The method according to claim 8, wherein the scaffold material is Matrigel.

10. A method for the preparation of an antibody, which comprises the following steps:
- preparing a hybridoma cell by a method as defined in any one of claims 1 to 9; and
- culturing the hybridoma cell to obtain the desired antibody.

11. A method according to claim 10, wherein the antibody binds to a membrane protein.

## Patentansprüche

1. Verfahren zur Herstellung einer Hybridomzelle, das folgende Schritte umfasst:
(1) Transplantieren von Brustkrebszellen in Brust, Brustdrüse, Knochen, Lunge oder Lymphknoten eines Versuchstiers, wobei die Brustkrebszellen wenigstens eine, ausgewählt aus der Gruppe bestehend aus MCF7-14, MDA-MB231, MDA- MB361 und MDA-MB435, ist;
(2) Halten des Tieres für 10 Wochen oder länger, um die Brustkrebszellen in das Versuchstier einzupflanzen; und
(3) Sammeln von Antikörper produzierenden Zellen von dem immunisierten Versuchstier, um diese mit Myelomzellen zu fusionieren,
dadurch Herstellen einer Hybridomzelle.

2. Verfahren nach Anspruch 1, wobei das Organ Brust oder Brustdrüse ist.

3. Verfahren nach Anspruch 1 oder 2, wobei die Brustdrüse die vierte Brustdrüse ist.

4. Verfahren nach einem der vorstehenden Ansprüche, wobei das Versuchstier ein Nagetier ist.

5. Verfahren nach einem der vorstehenden Ansprüche, wobei das Versuchstier ein immundefizientes Tier ist.

6. Verfahren nach einem der vorstehenden Ansprüche, wobei das Versuchstier eine BALB/c-nu/nu-Nacktmaus ist.

7. Verfahren nach einem der vorstehenden Ansprüche, wobei ein Gerüstmaterial für die Transplantation in Schritt (1) verwendet wird.

8. Verfahren nach Anspruch 7, wobei ein Bestandteil des Gerüstmaterials wenigstens einer, ausgewählt aus Laminin, Entactin, Kollagen, Fibrin, Agarose, Polyvinylalkohol, Polyethylenglokol, Polymilchsäure und Polyclycolsäure, ist.

9. Verfahren nach Anspruch 8, wobei das Gerüstmaterial Matrigel ist.

10. Verfahren zur Herstellung eines Antikörpers, das folgende Schritte umfasst:
- Herstellen einer Hybridomzelle durch ein Verfahren nach einem der Ansprüche 1 bis 9; und
- Kultivieren der Hybridomzelle, um den gewünschten Antikörper zu erzeugen.

11. Verfahren nach Anspruch 10, wobei der Antikörper an ein Membranprotein bindet.

## Revendications

1. Procédé de préparation d'une cellule d'hybridome, qui comprend les étapes suivantes :
(1) la transplantation de cellules de cancer du sein dans un sein, une glande mammaire, un os, un poumon ou un ganglion lymphatique d'un animal de laboratoire, les cellules de cancer du sein constituant au moins un élément choisi dans le groupe constitué par MCF7-14, MDA-MB231, MDA-MB361 et MDA-MB435 ;
(2) la conservation de l'animal pendant 10 semaines ou plus longtemps pour greffer les cellules de cancer du sein dans l'animal de laboratoire ; et
(3) le recueil de cellules produisant des anticorps provenant de l'animal de laboratoire immunisé pour les fusionner avec des cellules de myélome,
permettant ainsi de préparer une cellule d'hybridome.

2. Procédé selon la revendication 1, dans lequel l'organe est une glande du sein ou une glande mammaire.

3. Procédé selon la revendication 1 ou 2, dans lequel la glande mammaire est la quatrième glande mammaire.

4. Procédé selon l'une quelconque des revendications précédentes, dans lequel l'animal de laboratoire est un rongeur.

5. Procédé selon l'une quelconque des revendications précédentes, dans lequel l'animal de laboratoire est un animal immunodéficient.

6. Procédé selon l'une quelconque des revendications précédentes, dans lequel l'animal de laboratoire est une souris nude BALB/c-nu/nu.

7. Procédé selon l'une quelconque des revendications précédentes, dans lequel un matériau d'échafaudage est utilisé pour la transplantation dans l'étape (1).

8. Procédé selon la revendication 7, dans lequel un composant du matériau d'échafaudage est au moins un élément choisi parmi laminine, entactine, collagène, fibrine, agarose, alcool polyvinylique, polyéthylène glycol, acide polylactique et acide polyglycolique.

9. Procédé selon la revendication 8, dans lequel le matériau d'échafaudage est Matrigel.

10. Procédé de préparation d'une cellule d'anticorps, qui comprend les étapes suivantes :
- préparation d'une cellule d'hybridome par un procédé tel que défini dans l'une quelconque des revendications 1 à 9 ; et
- culture de la cellule d'hybridome pour obtenir l'anticorps désiré.

11. Procédé selon la revendication 10, dans lequel l'anticorps se lie à une protéine membranaire.
